# EUROPEAN PATENT APPLICATION

(11) **EP 4 534 181 A2**
(43) Date of publication of application: **09.04.2025**
(21) Application number: 24199384.9
(22) Date of filing: 10.09.2024
(51) Int. Cl.: B01D 53/14

(54) **CARBON DIOXIDE ABSORBING COMPOSITION, METHOD OF ABSORBING CARBON DIOXIDE AND METHOD OF SEPARATING CARBON DIOXIDE USING THE SAME**

(30) Priority: 12.09.2023 KR 20230120926
(71) Applicant: SK Innovation Co., Ltd., Seoul 03188 (KR)
(72) Inventor: KIM, Ji Su, 34124 Daejeon (KR); PARK, Eun Joon, 34124 Daejeon (KR); JUNG, Il Gu, 34124 Daejeon (KR); JEONG, Ji Su, 34124 Daejeon (KR); PYUN, Lim Ok, 34124 Deajeon (KR)
(74) Representative: Prüfer & Partner mbB Patentanwälte · Rechtsanwälte

(57) **Abstract**

Provided are a carbon dioxide absorbing composition including an ionic material containing a cyclic ammonium cation and a method of separating carbon dioxide using the same, and the carbon dioxide absorbing composition of the present disclosure has high thermal and chemical stability and excellent carbon dioxide absorption effect. In addition, the method of separating carbon dioxide using the carbon dioxide absorbing composition is very effective for desorption of carbon dioxide, and is a very economical method since it allows repeated use of the carbon dioxide absorbing composition.

## Description

### TECHNICAL FIELD

The following disclosure relates to a carbon dioxide absorbing composition comprising an ionic material comprising a cyclic ammonium cation and a method of separating carbon dioxide using the carbon dioxide absorbing composition.

### BACKGROUND

Due to the rapid progress of economic development and industrialization throughout the world, the use of fossil fuel as the main source of energy has also continuously increased. In particular, carbon dioxide (CO₂) accounts for the highest proportion of the main greenhouse gases formed in the process of burning fossil fuel to convert it into energy and is known to be closely related to a global warming problem. The global warming problem has intensified due to the increased and prolonged energy use and is receiving worldwide attention, and in order to solve the problem, the research area related to carbon dioxide becomes important.

As a method of separating carbon dioxide from exhaust gas from chemical plants, power plants, or large boilers and natural gas, an absorption method, an adsorption method, a separator method, a cryogenic separation method, and other methods are used. In particular, when the concentration of carbon dioxide emitted is low, an absorption or adsorption method is often used. Since the absorption or adsorption method may selectively separate only some gases absorbed or adsorbed on an absorbent or an adsorbent, it is industrially often used, but since an absorbent and an adsorbent are chemically modified during a separation process, they need to be replaced periodically.

When a solid adsorbent is used, the adsorbent is less chemically modified and it is favorable to apply the absorbent when an adsorbent replacement cycle is long.On the other hand, in a case of a liquid absorbent, it is easy to replace the absorbent, and thus, the absorption method is widely used in purification of exhaust gases or gas separation in large quantities, but the absorbent can be chemically modified during use.

As the carbon dioxide absorbent, an aqueous solution of amines such as monoethanolamine (MEA), N-methyldiethanolamine (MDEA), and diethanolamine (DEA) is most widely used, since a weakly alkaline amine absorbent forms a chemical bond with carbon dioxide which is an acidic gas, and when heat is applied, the bond is broken, carbon dioxide is degassed and recovered, and the absorbent may be regenerated.

However, the process using the aqueous solution of amines has several serious problems. In particular, problems such as irreversible decomposition of amine by a high temperature to break a chemical bond between carbon dioxide and an absorbent in a regeneration step of an amine absorbent, irreversible decomposition of amine by impurities such as SOₓ and NOₓ included in absorption gas, performance deterioration of an absorbent and supplement of an absorbent by the irreversible decomposition of amine, corrosion of an absorption device by amine itself or decomposition product, and contamination of gaseous carbon dioxide regenerated by the vapor pressure of an absorbent are being pointed out as disadvantages.

As an alternative thereto, an ionic liquid was suggested. An ionic liquid is composed of a cation such as quaternary ammonium, imidazolium, pyridinium, and phosphonium ions, and an inorganic anion such as [Cl]⁻, [Br]⁻, [I]⁻, [BF₄]⁻, [PF₆]⁻, and [Tf₂N]⁻, which has a relatively small size, or an organic anion such as [RCO₂]⁻ and [CO₃]²⁻. An ionic liquid is also referred to as a "designer solvent", due to its characteristics of allowing conversion into various characteristics by infinite combinations of cations, anions, functional groups, and the like.

Many studies on an ionic liquid based on an imidazolium cation and a [Cl]⁻, [BF₄]⁻, [PF₆]⁻, [CH₃SO₃]⁻, [CF₃BF₃]⁻, or [C₂F₅BF₃]⁻ anion and the syntheses thereon have been conducted. Among the ionic liquid, an ionic liquid including fluorine may be expected to have high carbon dioxide absorptive capacity, but is difficult to biodegrade, and thus, is environmentally harmful, and fluorinated anions are very expensive.

Therefore, there is a need to develop an absorbent which is an ionic material having optimal properties for separating carbon dioxide, being thermally stable so that there is no possibility of atmospheric emissions, being chemically stable to decrease atmospheric emissions of decomposition products, and desorbing carbon dioxide with excellent efficiency without causing corrosion of a device.

### SUMMARY

In some embodiments, the present disclosure provides a carbon dioxide absorbing composition which is not easily decomposed at a high temperature and has high stability in a carbon dioxide desorption process.

In some embodiments, the present disclosure provides a carbon dioxide absorbing composition which is thermally and/or chemically stable, so that atmospheric emissions of decomposition products may be decreased, and may save renewable energy with low vapor pressure.

In some embodiments, the present disclosure provides an environmentally friendly method of separating carbon dioxide economically by using the carbon dioxide absorbing composition.

In some embodiments, the present disclosure provides a method of capturing carbon dioxide.

In some embodiments, the present disclosure provides a method of reducing greenhouse gas emissions.

In some embodiments, a carbon dioxide absorbing composition comprises an ionic material comprising a cyclic ammonium cation represented by the following Chemical Formula 1:

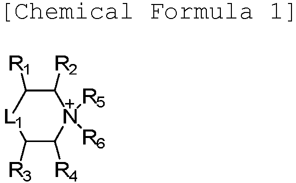

wherein
L₁ is a single bond, -O-, -NR₇-, or (C1-C8)alkylene,
R₁ to R₄ are each independently of one another hydrogen or (C1-C10)alkyl, and
R₅ to R₇ are each independently of one another (C1-C20)alkyl or hydroxy(Cl-C20)alkyl, or R₅ and R₇ may be bonded to each other to form a ring.

In some embodiments, the cyclic ammonium cation may be represented by the following Chemical Formula 2: 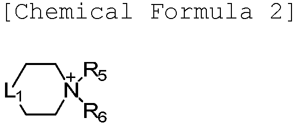 wherein
L₁ is a single bond, -O-, -NR₇-, or (C1-C5)alkylene, and
R₅ to R₇ are each independently of one another (C1-C10)alkyl or hydroxy(Cl-C10)alkyl, or R₅ and R₇ may be bonded to each other to form (C1-10)heterocycloalkyl.

In some embodiments, in Chemical Formula 2, L₁ may be a single bond, -O-, -NR₇-, or (C1-C3)alkylene, R₅ and R₆ each may be independently of each other (C1-C5)alkyl or hydroxy(C1-C5)alkyl, and R₇ may be (C1-C5)alkyl.

In some embodiments, in Chemical Formula 2, L₁ may be a single bond, -O-, -NR₇-, or methylene, R₅ and R₆ each may be independently of each other methyl, butyl, or hydroxy(C2-C3)alkyl, and R₇ may be methyl.

In some embodiments, the cyclic ammonium cation may be represented by the following Chemical Formula 3:

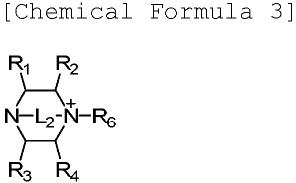

wherein
L₂ is (C1-C10) alkylene,
R₁ to R₄ are each independently of one another hydrogen or (C1-C5)alkyl, and
R₆ is (C1-C10)alkyl or hydroxy(Cl-C10)alkyl.

In some embodiments, in Chemical Formula 3, L₂ may be (C1-C5)alkylene, R₁ to R₄ may be independently of one another hydrogen or (C1-C3)alkyl, and R₆ may be (C1-C5)alkyl or hydroxy(C1-C5) alkyl.

In some embodiments, in Chemical Formula 3, L₂ may be ethylene, each R₁ to R₄ may be hydrogen, and R₆ may be butyl or propanol.

In some embodiments, the ionic material may further comprise a phenolate-based anion or an imidazolate-based anion.

In the carbon dioxide absorbing composition according to an exemplary embodiment, the ionic material may contain a phenolate-based anion represented by the following Chemical Formula 4 or an imidazolate-based anion represented by the following Chemical Formula 5:

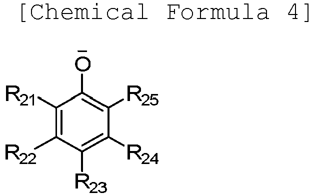

wherein
R₂₁ to R₂₅ are each independently of one another hydrogen, amino, hydroxy, nitro, carboxamido, C1-C10 alkyl, C1-C10 alkoxy, C1-C10 alkylcarbonyl, C1-C10 alkoxycarbonyl, C1-C10 alkylamino, diC1-C10 alkylamino, C1-C10 alkylaminoC1-C10 alkyl, or diC1-C10 alkylaminoC1-C10 alkyl, and
the amino, hydroxy, carboxamido, alkyl, alkoxy, alkylcarbonyl, alkoxycarbonyl, alkylamino, dialkylamino, alkylaminoalkyl, and dialkylaminoalkyl may be substituted by one or two or more substituents selected from halogen, cyano, amino, nitro, C1-C10 alkyl, C1-C10 alkoxy, C1-C10 alkylcarbonyl, and C1-C10 alkoxycarbonyl, 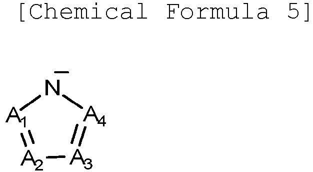
wherein
A₁ to A₄ are independently of one another CR or N,
at least one of A₁ to A₄ is N, and
R is hydrogen, amino, C1-C10 alkyl, C1-C10 alkoxy, C1-C10 alkylcarbonyl, C1-C10 alkoxycarbonyl, C1-C10 alkylamino, diC1-C10 alkylamino, C1-C10 alkylcarbonylamino, C1-C10 alkylaminoC1-C10 alkyl, or diC1-C10 alkylaminoC1-C10 alkyl, or two or more R's may be connected to each other to form a ring.

In some embodiments, the carbon dioxide absorbing composition may further comprise water.

In some embodiments, the carbon dioxide absorbing composition may further comprise one or more solvents other than water.

In some embodiments, the carbon dioxide absorbing composition may have a carbon dioxide absorption equivalent represented by the following Equation 1 of 0.6 or more: Carbon dioxide absorption equivalent = (moles of absorbed carbon dioxide) / (moles of ionic material in absorbing composition)

In some embodiments, the carbon dioxide absorbing composition may further comprise one or more amine absorbents.

In some embodiments, the carbon dioxide absorbing composition further including one or more amine absorbents may further comprise water.

In some embodiments, the carbon dioxide absorbing composition comprising one or more amine absorbants may further comprise one or more solvents other than water.

In another aspect of the present disclosure, there is provided a method of absorbing carbon dioxide comprising: reacting carbon dioxide and a phenolate-based anion or an imidazolate-based anion to respectively form a carbonate or a carbamate in an ionic material with a cyclic ammonium cation, thereby obtaining a carbon dioxide absorbing composition, and additionally physically absorbing carbon dioxide in the carbon dioxide absorbing composition including the ionic material.

According to an exemplary embodiment of the method of absorbing carbon dioxide, reacting carbon dioxide involves the cyclic ammonium cation to form the ionic material by following the Reaction Formula 1 to form the carbonate and Reaction Formula 2 to form the carbamate respectively specified below:

[Reaction Formula 1]: CO₂ + [heterocycle-N⁺][phenolate-O⁻] → [heterocycle-N⁺][phenolate-O-CO₂⁻]

[Reaction Formula 2] CO₂ + [heterocycle-N⁺][imidazolate-N⁻] --+ [heterocycle-N⁺][imidazolate-N-CO₂⁻],

wherein [heterocycle-N⁺] denotes the cyclic ammonium cation compound, optionally the cyclic ammonium cation of [Chemical Formula 1], [Chemical Formula 2] or [Chemical Formula 3], and wherein [phenolate-O⁻] denotes a phenolate-based anion, preferably represented by the [Chemical Formula 4], and [imidazolate-N⁻] denotes an imidazolate-based anion, preferably represented by [Chemical Formula 5] of the exemplary embodiments of the first general aspect, respectively.

In some embodiments of the method of absorbing carbon dioxide, the carbon dioxide absorbing composition is as defined in the first general aspect specified above.

In some embodiments, a method of separating carbon dioxide compriese: a first step of bringing a carbon dioxide absorbing composition as disclosed herein into contact with a mixture comprising carbon dioxide; or carrying out the method of absorbing carbon dioxide according to an exemplary embodiment of the present disclosure; and a second step of heat treating the carbon dioxide absorbing composition to desorb carbon dioxide attached to the absorbing composition.

In some embodiments, the first step may be performed under the conditions of 20 to 80°C.

In some embodiments, the second step may be performed under the conditions of 70 to 150°C.

In some embodiments, in the method of separating carbon dioxide, when the first step and the second step are a unit process, carbon dioxide may be continuously separated by repeating the unit process twice or more.

Other features and aspects will be apparent from the following detailed description, and the claims.

### DETAILED DESCRIPTION OF EMBODIMENTS

Hereinafter, the present disclosure will be described in detail. Terms used in the present specification should be interpreted as having the meaning commonly understood by a person skilled in the art to which the present disclosure pertains, unless otherwise defined. Examples of the present specification are for a person skilled in the art to easily understand and carry out the present disclosure, descriptions which may obscure the gist of the present disclosure in the examples may be omitted, and the present disclosure is not limited by the examples.

The singular form used in the present specification may be intended to also include a plural form, unless otherwise indicated in the context. As used herein, the singular form of "a", "an", and "the" include plural referents unless the context clearly states otherwise.

In addition, the numerical range used in the present specification includes all values within the range including the lower limit and the upper limit, increments logically derived in a form and span of a defined range, all double limited values, and all possible combinations of the upper limit and the lower limit in the numerical range defined in different forms. Unless otherwise defined in the present specification, values which may be outside a numerical range due to experimental error or rounding off of a value are also included in the defined numerical range.

For the purposes of this specification, unless otherwise indicated, all numbers expressing quantities of ingredients, reaction conditions, dimensions, physical characteristics, and so forth used in the specification and claims are to be understood as being modified in all instances by the term "about." Hereinafter, unless otherwise particularly defined in the present specification, "about" may be considered as a value within 30%, 25%, 20%, 15%, 10%, 5%, 3%, 2%, 1%, 0.5%, 0.1%, 0.05% or 0.01 of a stated value. Unless indicated to the contrary, the numerical parameters set forth in the following specification and attached claims are approximations that can vary depending upon the desired properties sought to be obtained by the present invention.

Notwithstanding that the numerical ranges and parameters setting forth the broad scope of the invention are approximations, the numerical values set forth in the specific examples are reported as precisely as possible. Any numerical value, however, inherently contains certain errors necessarily resulting from the standard deviation found in their respective testing measurements.

In the present specification, the terms such as "comprise", "contain", "have", and "provided with" mean that there is a characteristic or a constituent element described in the specification, and as long as it is not particularly limited, a possibility of adding one or more other characteristics or constituent elements is not excluded in advance.

The term "single bond" in the present specification refers to a case in which there is no atom in the corresponding area. For example, in a structure of X-Y-Z, when Y is a single bond, X and Z is directly bonded to form a structure of X-Z.

In some embodiments, there is provided a carbon dioxide absorbing composition comprising an ionic material comprising a cyclic ammonium cation represented by the following Chemical Formula 1:

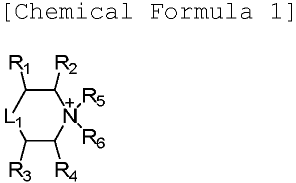

wherein
L₁ is a single bond, -O-, -NR₇-, or (C1-C8)alkylene,
R₁ to R₄ are each independently of one another hydrogen or (C1-C10)alkyl, and
R₅ to R₇ are each independently of one another (C1-C20)alkyl or hydroxy(Cl-C20)alkyl, or R₅ and R₇ may be bonded to each other to form a ring.

In some embodiments, the carbon dioxide absorbing composition is thermally and/or chemically stable, and when it is applied to a process, it has a low loss of the carbon dioxide absorbing composition by decomposition. In some embodiments, since the carbon dioxide absorbing composition with the ionic material is not easily decomposed in a desorption process under basic conditions at a high temperature, they may reduce periodic supplementation of the absorbing composition when they are applied to the process, and thus, are economical.

Chemical stability at high temperature is particularly enhanced when the spiro ammonium cation is combined with a phenolate-based anion or an imidazolate-based anion to form the ionic material of the carbon dioxide absorbing composition.

An amine absorbent (e.g., monoethanolamine (MEA)) used in the conventional carbon dioxide absorbing composition has a benefit of a rapid reaction rate with carbon dioxide, but also has a problem of a high decomposition ratio after a carbon dioxide desorption process. However, when the carbon dioxide absorbing composition(s) according to the present disclosure comprises an ionic material comprising a cyclic ammonium cation having a specific structure and a phenolate-based anion and/or an imidazolate-based anion, it may decrease a decomposition ratio after the carbon dioxide desorption process as compared with a conventional amine absorbent.

Since the ionic material(s) of the present disclosure has lower corrosiveness than the conventional amine compound, a carbon dioxide absorbing composition comprising an ionic material at a high concentration may be prepared by using the ionic material, and the carbon dioxide absorbing composition may have significantly improved carbon dioxide absorption performance as compared with the conventional carbon dioxide absorbing composition including amine compounds.

In some embodiments, the cyclic ammonium cation of the present disclosure may be represented by the following Chemical Formula 2:

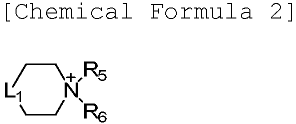

wherein
L₁ is a single bond, -O-, -NR₇-, or (C1-C5)alkylene, and
R₅ to R₇ are each independently of one another (C1-C10)alkyl or hydroxy(Cl-C10)alkyl, or R₅ and R₇ may be bonded to each other to form (C1-10)heterocycloalkyl.

In some embodiments, in Chemical Formula 2, L₁ may be a single bond, -O-, -NR₇-, or (C1-C3)alkylene, R₅ and R₆ each may be independently of each other (C1-C5)alkyl or hydroxy(C1-C5)alkyl, and R₇ may be (C1-C5)alkyl.

In some embodiments,, in Chemical Formula 2, L₁ may be a single bond, -O-, -NR₇-, or methylene, R₅ and R₆ may be independently of each other methyl, butyl, or hydroxy(C2-C3)alkyl, and R₇ may be methyl.

In some embodiments, the cyclic ammonium cation of the present disclosure may be represented by the following Chemical Formula 3:

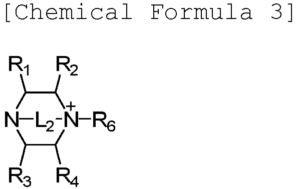

wherein
L₂ is (C1-C10)alkylene,
R₁ to R₄ are each independently of one another hydrogen or (C1-C5)alkyl, and
R₆ is (C1-C10)alkyl or hydroxy(Cl-C10)alkyl.

In some embodiments, in Chemical Formula 3, L₂ may be (C1-C5)alkylene, R₁ to R₄ each may be independently of one another hydrogen or (C1-C3)alkyl, and R₆ may be (C1-C5)alkyl or hydroxy(C1-C5)alkyl.

In some embodiments, in Chemical Formula 3, L₂ may be ethylene, R₁ to R₄ may be hydrogen, and R₆ may be butyl or propanol.

In some embodiments, in the carbon dioxide absorbing composition of the present disclosure, the ionic material may further comprise a phenolate-based anion represented by the following Chemical Formula 4 amd/or an imidazolate-based anion represented by the following Chemical Formula 5: 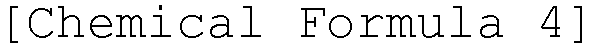 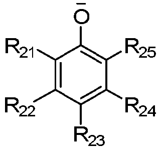 wherein
R₂₁ to R₂₅ are each independently of one another hydrogen, amino, hydroxy, nitro, carboxamido, C1-C10 alkyl, C1-C10 alkoxy, C1-C10 alkylcarbonyl, C1-C10 alkoxycarbonyl, C1-C10 alkylamino, diC1-C10 alkylamino, C1-C10 alkylaminoC1-C10 alkyl, or diC1-C10 alkylaminoC1-C10 alkyl, and
the amino, hydroxy, carboxamido, alkyl, alkoxy, alkylcarbonyl, alkoxycarbonyl, alkylamino, dialkylamino, alkylaminoalkyl, and dialkylaminoalkyl may be substituted by one or two or more substituents selected from halogen, cyano, amino, nitro, C1-C10 alkyl, C1-C10 alkoxy, C1-C10 alkylcarbonyl, and C1-C10 alkoxycarbonyl,

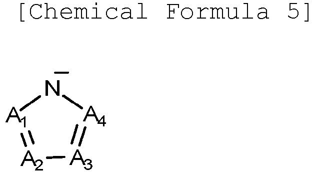

   wherein
A₁ to A₄ are each independently of one another CR or N,
at least one of A₁ to A₄ is N, and
R is hydrogen, amino, C1-C10 alkyl, C1-C10 alkoxy, C1-C10 alkylcarbonyl, C1-C10 alkoxycarbonyl, C1-C10 alkylamino, diC1-C10 alkylamino, C1-C10 alkylcarbonylamino, C1-C10 alkylaminoC1-C10 alkyl, or diC1-C10 alkylaminoC1-C10 alkyl, or two or more R's may be connected to each other to form a ring.

In some embodiments, the carbon dioxide absorbing composition may comprise 20 to 80 wt%, or 25 to 77 wt%, or 30 to 75 wt% of the ionic material, based on the total weight of the composition.

In some embodiments, the carbon dioxide absorbing composition may further comprise water, and/or one or more solvents other than water. The solvent other than water may be a nonaqueous solvent, and for example, may be one or more selected from ethylene glycol, propylene glycol, methyl glycol, methyl isopropyl carboxylate, methyl diethyl carboxylate, triethylene glycol, dimethyl sulfonate, and diethyl sulfonate, and may be ethylene glycol.

In some embodiments, the carbon dioxide absorbing composition may comprise 20 to 80 wt%, or23 to 75 wt%, or 25 to 70 wt% of water, a solvent other than water, or a mixed solvent of water and a solvent other than water (such as a nonaqueous solvent).

In some embodiments, the carbon dioxide absorbing composition may have a carbon dioxide absorption equivalent represented by the following Equation 1 of 0.6 or more, or 0.6 to 3.0: Carbon dioxide absorption equivalent = (moles of absorbed carbon dioxide) / (moles of ionic material in absorbing composition).

In the carbon dioxide absorption mechanism of the ionic material comprising the cyclic ammonium cation and the phenolate-based anion and/or the imidazolate-based anion, a better carbon dioxide absorption equivalent may be shown by not only a chemical absorption method in which carbon dioxide and the phenolate-based anion and/or the imidazolate-based anion of the absorbing composition are reacted to form a carbonate and a carbamate represented by Reaction Formulae 1 and 2 but also a physical absorption method of trapping carbon dioxide in "free volume" of the carbon dioxide absorbing composition comprising the ionic material:

[Reaction Formula 1] CO₂ + [heterocycle-N⁺][phenolate-O⁻] → [heterocycle-N⁺][phenolate-O-CO₂⁻]

[Reaction Formula 2] CO₂ + [heterocycle-N⁺] [imidazolate-N⁻] → [heterocycle-N⁺][imidazolate-N-CO₂⁻]

Accordingly, in an aspect of the present disclosure, a new concept of carbon dioxide absorption is embodied by a method in which carbon dioxide and a phenolate-based anion or a imidazolate-based anion are reacted to form a carbonate and a carbamate of an absorbing composition in which carbon dioxide is chemically bound in an ionic material, and optionally in addition by a physical absorption method of trapping carbon dioxide in "free volume" of the carbon dioxide absorbing composition including the ionic material.

In specific embodiments of this new concept and aspect of the present disclosure, the reaction of carbon dioxide involves the cyclic ammonium cation disclosed herein and follows the Reaction Formulae 1 and 2 specified above.

In this new concept and further aspect of the present disclosure too, chemical stability at high temperature is particularly enhanced when the cyclic ammonium cation is combined with a phenolate-based anion or an imidazolate-based anion to form the ionic material of the carbon dioxide absorbing composition.

In a further embodiment of this new concept and aspect of the present disclosure, the carbon dioxide absorption composition is defined as in the previously described aspect of the present disclosure.

The carbon dioxide absorbing composition comprising the ionic material may show a significantly improved effect on the absorption equivalent as compared with a conventional carbon dioxide absorbing composition of amines by the use of the cyclic ammonium cation material having a specific structure, in particular when combined with the phenolate-based anion or the imidazolate-based anion.

In some embodiments, the carbon dioxide absorbing composition may further comprise one or more amine absorbents, for example 1 to 5 or 1 to 3 amine absorbents. In some embodiments, the amine absorbent may be selected from monoethanolamine (MEA), N-methyldiethanolamine (MDEA), diethanolamine (DEA), triethanolamine (TEA), 2-amino-2-methyl-1-propanol (AMP), and/or piperazine (PZ).

In some embodiments, the carbon dioxide absorbing composition comprising the amine absorbent may further comprise water, and/or one or more solvents other than water in amounts as disclosed herein above.

In some embodiments, since the carbon dioxide absorbing composition comprising the ionic material has a low decrease rate of the carbon dioxide absorbing composition when it is stored for a long time at a heat treatment temperature for desorbing carbon dioxide which is attached to the absorbing composition, performance of the carbon dioxide absorbing composition may be maintained stable even after the carbon dioxide desorption process. The decrease rate of the carbon dioxide absorbing composition represented by the following Equation 2 may be 5% or less, or 4.5% or less, 3% or less, 2% or less, and more preferably 1.5% or less: Decrease rate of carbon dioxide absorbing composition = 100 - {(moles of ionic material after heat treatment) / (moles of ionic material before heat treatment)} × 100

In some embodiments, a method of separating carbon dioxide using the carbon dioxide absorbing composition is provided. The method of separating carbon dioxide may comprise: a first step of bringing a carbon dioxide absorbing composition into contact with a mixture comprising carbon dioxide; and a second step of heat treating the carbon dioxide absorbing composition to desorb carbon dioxide attached to the absorbing composition.

In some embodiments, in the method of separating carbon dioxide, decomposition of the carbon dioxide absorbing composition is inhibited in the second step of desorbing carbon dioxide under basic conditions at a high temperature, thereby decreasing the decrease rate of the absorbing composition after desorbing carbon dioxide. Accordingly, the method of separating carbon dioxide has a small loss of the absorbing composition depending on the number of processes as compared with a method of absorbing carbon dioxide using the conventional carbon dioxide absorbing composition of amines, and thus, the amount of the absorbing composition which should be replenished after each process may be decreased and the process may be operated more economically.

In some embodiments, the first step of the method of separating carbon dioxide may be performed under the conditions of 20 to 80°C, or 25°C to 65°C, or 30°C to 50°C, but is not limited thereto.

In some embodiments, the second step of the method of separating carbon dioxide may be performed under the conditions of 70 to 150°C, or 80°C to 140°C, or 90°C to 130°C, but is not limited thereto.

In some embodiments, in the method of separating carbon dioxide according to the present disclosure, when the first step and the second step are a unit process, carbon dioxide may be continuously separated by repeating the unit process twice or more.

In some embodiments, the carbon dioxide absorbing composition according to the present disclosure is thermally and chemically stable, and/or has a low loss of the carbon dioxide absorbing composition by decomposition. Therefore, in some embodiments, in the method of separating carbon dioxide using the carbon dioxide absorbing composition, atmospheric emissions of by-products by decomposition of the absorbing composition are low for the reason described above, and may be a very environmentally friendly method.

In some embodiments, the method of separating carbon dioxide using the carbon dioxide absorbing composition has a low decomposition rate of the carbon dioxide absorbing composition after a carbon dioxide desorption process, and thus, allows repeated use of the absorbing composition and is a very economical method.

In some embodiments, a method of capturing carbon dioxide is provided, comprising contacting carbon dioxide with a carbon dioxide absorbing composition comprising an ionic material comprising a cyclic ammonium cation represented by the following Chemical Formula 1:

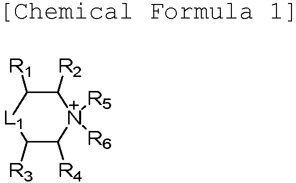

wherein
L₁ is a single bond, -O-, -NR₇-, or (C1-C8)alkylene,
R₁ to R₄ are each independently of one another hydrogen or (C1-C10)alkyl, and
R₅ to R₇ are each independently of one another (C1-C20)alkyl or hydroxy(C1-C20)alkyl, or R₅ and R₇ may be bonded to each other to form a ring whereby the carbon dioxide absorbing composition captures the carbon dioxide.

In some embodiments, a method of reducing greenhouse gas emissions comprising carbon dioxide is provided, comprising: contacting the greenhouse gas emissions with a carbon dioxide absorbing composition comprising an ionic material comprising a cyclic ammonium cation represented by the following Chemical Formula 1:

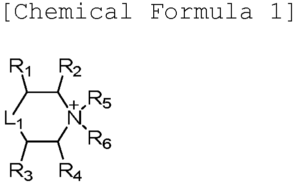

wherein
L₁ is a single bond, -O-, -NR₇-, or (C1-C8)alkylene,
R₁ to R₄ are each independently of one another hydrogen or (C1-C10)alkyl, and
R₅ to R₇ are each independently of one another (C1-C20)alkyl or hydroxy(Cl-C20)alkyl, or R₅ and R₇ may be bonded to each other to form a ring whereby the carbon dioxide absorbing composition captures carbon dioxide from the greenhouse gas emissions.

Hereinafter, some embodiments of the carbon dioxide absorbing composition comprising the ionic material comprising the cyclic ammonium cation and the method of separating carbon dioxide using the same will be described in more detail by the specific examples.

### [Preparation Example 1] Preparation of ionic material IC-1

40 mmol of a chloride compound or a cyclic ammonium cation shown in "IC-1" of the following Table 1 and 40 mmol of a sodium compound of a phenolate anion also shown in "IC-1" of the following Table 1 were added, 40 mL of ethanol was added, and stirring was performed at room temperature for 2 hours. The produced sodium chloride solid was removed by filtration, and drying under vacuum was performed at 50°C for 15 hours to obtain an ionic material IC-1. The obtained ionic material IC-1 was measured by NMR, and the results are shown in Table 1.

### [Preparation Examples 2 to 8] Preparation of ionic materials IC-2 to IC-8

Ionic materials IC-2 to IC-8 were prepared in the same manner as in Preparation Example 1, except that the chloride compounds of cyclic ammonium cations shown in "IC-2" to "IC-8" of the following Table 1 and the sodium compound of phenolate anions or an imidazolate anion also shown in "IC-2" to "IC-8" of the following Table 1 were used instead of the chloride cation compound and the anion sodium compound used in Preparation Example 1. The obtained ionic materials were measured by NMR, and the results are shown in Table 1.

**[Table 1]**

| | Ionic material | NMR results |
|---|---|---|
| Preparation Example 1 | 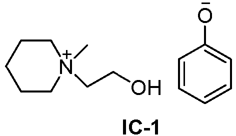 | ¹H NMR (500 MHz), D₂O, 25°C: δ=7.14(t, 2H), 6.60(m, 3H), 3.99(t, 2H), 3.43(t, 2H), 3.37(m, 2H), 3.30(m, 2H), 3.05(s, 3H), 1.83(m, 4H), 1.61(m, 2H) |
| Preparation Example 2 | 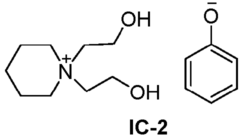 | ¹H NMR (500 MHz), D₂O, 25°C: δ=7.15(t, 2H), 6.61(m, 3H), 3.98(t, 4H), 3.56(t, 4H), 3.43(t, 4H), 1.85(m, 4H), 1.64(m, 2H) |
| Preparation Example 3 | 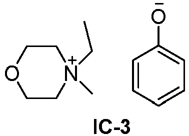 | ¹H NMR (500MHz), D₂O, 25°C: δ=7.18(t, 2H), 6.67(m, 3H), 4.01(t, 4H), 3.42(m, 6H), 3.10(t, 3H), 1.37(t, 3H) |
| Preparation Example 4 | 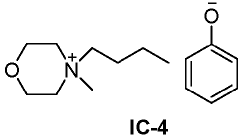 | ¹H NMR (500MHz), D₂O, 25°C: δ=7.18(t, 2H), 6.68(m, 3H), 3.99(t, 4H), 3.42(m, 6H), 3.11(s, 3H), 1.74(m, 2H), 1.38(m, 2H), 0.97(m, 3H) |
| Preparation Example 5 | 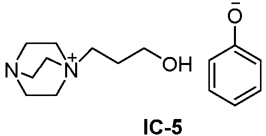 | ¹H NMR (500 MHz), D₂O, 25°C: δ=7.01(t, 2H), 6.49(d, 2H), 6.43(t, 1H), 3.50 (m, 2H) 3.15(m, 8H), 3.12(m, 6H), 1.93(m, 2H) |
| Preparation Example 6 | 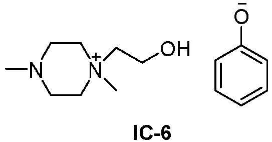 | ¹H NMR (500 MHz), D₂O, 25°C: δ=6.90(t, 2H), 6.42(d, 2H), 6.33(t, 1H), 3.80(m, 2H) 3.29(m, 4H), 3.23(m, 2H), 2.92(s, 3H), 2.59(m, 4H), 2.15(s, 3H) |
| Preparation Example 7 | 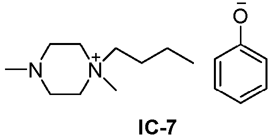 | ¹H NMR (500 MHz), D₂O, 25°C: δ=6.95(t, 2H), 6.45(d, 2H), 6.39(t, 1H), 3.16(m, 6H) 2.92(s, 3H), 2.61(m, 4H), 2.17(s, 3H), 1.51(m, 2H), 1.20(m, 2H), 0.80(t, 3H) |
| Preparation Example 8 | 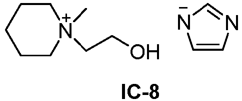 | ¹H NMR (500 MHz), D₂O, 25°C: δ=7.61(s, 1H), 7.00(s, 2H), 3.87(t, 2H), 3.27(m, 4H), 3.19(m, 2H), 2.96(s, 3H), 1.74(m, 4H), 1.52(m, 2H) |

### [Examples 1 to 10]

The carbon dioxide absorbing compositions of Examples 1 to 10 including the ionic materials of Preparation Examples 1 to 8 and solvents having the compositions of Table 2 were prepared.

The ionic material and the solvent were added according to wt% regardless of the addition order, and mixing was performed at 20 to 40°C for 5 to 60 minutes to prepare the carbon dioxide absorbing compositions of Examples 1 to 10.

**[Table 2]**

| | Ionic material (wt%) | Solvent (wt%) |
|---|---|---|
| Example 1 | Preparation Example 1 (IC-1) (30%) | Water (70%) |
| Example 2 | Preparation Example 2 (IC-2) (30%) | Water (70%) |
| Example 3 | Preparation Example 3 (IC-3) (30%) | Water (70%) |
| Example 4 | Preparation Example 4 (IC-4) (30%) | Water (70%) |
| Example 5 | Preparation Example 5 (IC-5) (30%) | Water (70%) |
| Example 6 | Preparation Example 6 (IC-6) (30%) | Water (70%) |
| Example 7 | Preparation Example 7 (IC-7) (30%) | Water (70%) |
| Example 8 | Preparation Example 8 (IC-8) (30%) | Water (70%) |
| Example 9 | Preparation Example 1 (IC-1) (45%) | Water (45%), ethylene glycol (10%) |
| Example 10 | Preparation Example 8 (IC-8) (45%) | Water (45%), ethylene glycol (10%) |

### [Comparative Example 1] Preparation of carbon dioxide absorbing composition

The carbon dioxide absorbing composition of Comparative Example 1 including amine and a solvent was prepared with the composition of the following Table 3.

The amine and the solvent were added according to the weight ratio regardless of the addition order, and mixing was performed at 20 to 40°C for 5 to 60 minutes to prepare the carbon dioxide absorbing composition of Comparative Example 1.

**[Table 3]**

| | Amine (wt%) | Solvent (wt%) |
|---|---|---|
| Comparative Example 1 | 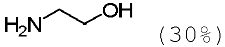 | Water (70%) |

### [Experimental Example 1] Evaluation of carbon dioxide absorption performance

In order to evaluate the carbon dioxide absorption performance of the carbon dioxide absorbing compositions according to Examples 1 to 8 and Comparative Example 1, the carbon dioxide absorption performance was measured using a vapor liquid equilibrium (VLE) apparatus manufactured according to a reference (Applied Chemistry for Engineering, 2010, 21(3), 284-290). As the vapor liquid equilibrium apparatus, an apparatus including a cylinder for storing carbon dioxide (150 mL), a constant temperature water bath, a stainless steel reactor equipped with a thermometer (73 mL), an electronic pressure gauge, and an agitator was used. At this time, absorption ability was measured by maintaining a constant temperature of 40°C in the cylinder and the reactor using a constant temperature water bath and a heating block, respectively. A measurement error range of the reactor was ±0.1°C and ±0.01 bar.

Evaluation of carbon dioxide absorption performance was performed specifically by the following method. First, the insides of a cylinder for storing carbon dioxide and an absorption reactor were sufficiently replaced with nitrogen, and the cylinder for storing carbon dioxide was filled with carbon dioxide and maintained at 40°C under 1 bar. Next, the carbon dioxide absorbing compositions (6.0 g) of Examples 1 to 8 and Comparative Example 1 were added into the reactor, which was maintained at 40°C, a valve connecting the cylinder and the reactor was opened, and a pressure was measured after reaching absorption equilibrium. An equilibrium pressure was measured every 30 minutes, and the process was repeated until there was no change in pressure between the cylinder and the reactor.

An ideal gas equation was used to calculate the number of moles of absorbed carbon dioxide depending on pressure change. The number of moles of the absorbed carbon dioxide was used to analyze the carbon dioxide absorption equivalent calculated by the following Equation 3, which is shown in the following Table 4: Carbon dioxide absorption equivalent = (moles of absorbed carbon dioxide) / (moles of ionic material in absorbing composition)

**[Table 4]**

| | Carbon dioxide absorption equivalent (mol/mol) |
|---|---|
| Example 1 | 1.06 |
| Example 2 | 0.85 |
| Example 3 | 1.03 |
| Example 4 | 1.01 |
| Example 5 | 0.81 |
| Example 6 | 0.61 |
| Example 7 | 0.64 |
| Example 8 | 0.94 |
| Comparative Example 1 | 0.59 |

### [Experimental Example 2] Thermal stability evaluation

In order to evaluate the thermal stability of the carbon dioxide absorbing compositions according to Examples 9 and 10 and Comparative Example 1, carbon dioxide was bubbled into the absorbing compositions for 30 minutes or more at a speed of 100 cc/min to sufficiently absorb carbon dioxide. 10.0 g of the composition to which carbon dioxide was absorbed was added to a stainless steel container (50 mL), which was heat treated by being stored in an oven maintained at a constant temperature of 130°C for 4.5 days, and the decrease rate of the carbon dioxide absorbing composition calculated by the following Equation 4 is shown in the following Table 5: Decrease rate of carbon dioxide absorbing composition = 100 - {(moles of ionic material after heat treatment) / (moles of ionic material before heat treatment)} × 100

**[Table 5]**

| | Average decrease rate (%) |
|---|---|
| Example 9 | 3.5 |
| Example 10 | 3.7 |
| Comparative Example 1 | 5.4 |

As shown in Table 4, it was found that the carbon dioxide absorption equivalents of the examples were all 0.6 or more, which showed improved carbon dioxide absorption performance as compared with the comparative example.

In addition, as shown in Table 5, it was found that the decrease rate of the ionic material of the Examples 9 and 10 was 4.0% or less, and the compositions had excellent stability at a high temperature. However, the comparative example to which monoethanolamine which is widely used as a carbon dioxide absorbing composition of amines was introduced showed a higher decrease rate than Examples 9 and 10, and it was found from the results that the Examples of the present disclosure showed significantly improved stability in the carbon dioxide absorption process.

Since the carbon dioxide absorbing composition comprising the ionic material comprising the cyclic ammonium cation and the phenolate-based anion or the imidazolate-based anion according to the present disclosure had improved stability under the basic environment at a high temperature, the problems of desorption and reuse restriction occurring in the conventional carbon dioxide absorbing composition using monoethanolamine were improved and it was very easy to apply a process for mass production.

In addition, the method of separating carbon dioxide using the carbon dioxide absorbing composition was more effective for desorption of carbon dioxide than a method using the carbon dioxide absorbing composition of amines, and was a very economical method since it allowed repeated use of the carbon dioxide absorbing composition.

The carbon dioxide absorbing composition according to the present disclosure is thermally and/or chemically stable to reduce atmospheric emissions of decomposition products when it is applied to a process, and/or has thermal stability since it is not easily decomposed even under basic conditions at high temperature as compared with a conventional carbon dioxide absorbing composition of amines.

In addition, since the carbon dioxide absorbing composition according to the present disclosure can have low corrosiveness as compared with the conventional carbon dioxide absorbing composition of amines, a carbon dioxide absorbing composition including an ionic material at a high concentration may be prepared, whereby significantly improved absorption performance may be shown. In addition, the carbon dioxide absorbing composition of the present disclosure can allow easy desorption of carbon dioxide even with low renewable energy and is very effective for absorbing and desorbing carbon dioxide, as compared with the conventional carbon dioxide absorbing composition of amines.

The method of separating carbon dioxide using the carbon dioxide absorbing composition according to the present disclosure can allow improved absorption performance and/or stable process operation, and thus, may be easily industrially applied in a very economical and environmentally friendly manner.

Hereinabove, although the present disclosure has been described by specific matters, and limited examples and comparative example, they have been provided only for assisting the entire understanding of the present disclosure, and the present disclosure is not limited to the implementations, and various modifications and changes may be made by those skilled in the art to which the present disclosure pertains from the description.

Therefore, the present disclosure should not be limited to the above-described exemplary embodiments, in keeping with the scope and spirit of the disclosure.

## Claims

1. A carbon dioxide absorbing composition comprising an ionic material comprising a cyclic ammonium cation represented by the following Chemical Formula 1: 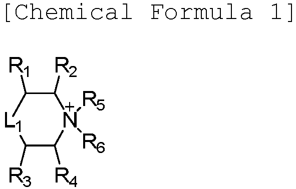 wherein
L₁ is a single bond, -O-, -NR₇-, or (C1-C8)alkylene,
R₁ to R₄ are each independently of one another hydrogen or (C1-C10)alkyl, and
R₅ to R₇ are each independently of one another (C1-C20)alkyl or hydroxy(Cl-C20)alkyl, or R₅ and R₇ may be bonded to each other to form a ring.

2. The carbon dioxide absorbing composition of claim 1, wherein the cyclic ammonium cation is represented by the following Chemical Formula 2: 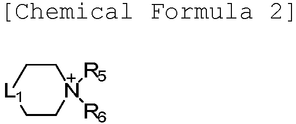 wherein
L₁ is a single bond, -O-, -NR₇-, or (C1-C5) alkylene, and
R₅ to R₇ are each independently of one another (C1-C10)alkyl or hydroxy(Cl-C10)alkyl, or R₅ and R₇ may be bonded to each other to form (C1-10)heterocycloalkyl,
preferably wherein L₁ a single bond, -O-, -NR₇-, or (C1-C3)alkylene, R₅ and R₆ is independently of each other (C1-C5)alkyl or hydroxy(C1-CS)alkyl, and R₇ is (C1-C5)alkyl,
more preferably wherein L₁ is a single bond, -O-, - NR₇-, or methylene,
R₅ and R₆ are each independently of each other methyl, butyl, or hydroxy(C2-C3)alkyl, and
R₇ is methyl.

3. The carbon dioxide absorbing composition of claim 1,
wherein the cyclic ammonium cation is represented by the following Chemical Formula 3: 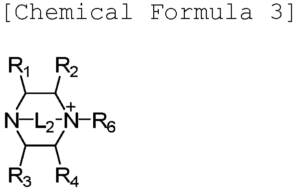
wherein
L₂ is (C1-C10)alkylene,
R₁ to R₄ are each independently of one another hydrogen or (C1-C5)alkyl, and
R₆ is (C1-C10)alkyl or hydroxy(C1-C10)alkyl,
preferably wherein L₂ is (C1-C5)alkylene,
R₁ to R₄ are independently of one another hydrogen or (C1-C3)alkyl, and
R₆ is (C1-C5)alkyl or hydroxy(C1-CS)alkyl,
more preferably wherein L₂ is ethylene,
R₁ to R₄ are hydrogen, and
R₆ is butyl or propanol.

4. The carbon dioxide absorbing composition of any one of the preceding claims, wherein the ionic material further comprises a phenolate-based anion and/or an imidazolate-based anion, preferably wherein the phenolate-based anion represented by the following Chemical Formula 4 and/or wherein the imidazolate-based anion is represented by the following Chemical Formula 5: 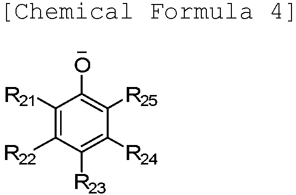 wherein
R₂₁ to R₂₅ are each independently of one another hydrogen, amino, hydroxy, nitro, carboxamido, C1-C10 alkyl, C1-C10 alkoxy, C1-C10 alkylcarbonyl, C1-C10 alkoxycarbonyl, C1-C10 alkylamino, diC1-C10 alkylamino, C1-C10 alkylaminoC1-C10 alkyl, or diC1-C10 alkylaminoC1-C10 alkyl, and
the amino, hydroxy, carboxamido, alkyl, alkoxy, alkylcarbonyl, alkoxycarbonyl, alkylamino, dialkylamino, alkylaminoalkyl, and/or dialkylaminoalkyl may be substituted by one or two or more substituents selected from halogen, cyano, amino, nitro, C1-C10 alkyl, C1-C10 alkoxy, C1-C10 alkylcarbonyl, and C1-C10 alkoxycarbonyl, 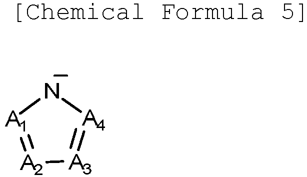
wherein
A₁ to A₄ are independently of one another CR or N,
at least one of A₁ to A₄ is N, and
R is hydrogen, amino, C1-C10 alkyl, C1-C10 alkoxy, C1-C10 alkylcarbonyl, C1-C10 alkoxycarbonyl, C1-C10 alkylamino, diC1-C10 alkylamino, C1-C10 alkylcarbonylamino, C1-C10 alkylaminoC1-C10 alkyl, or diC1-C10 alkylaminoC1-C10 alkyl, or two or more R's may be connected to each other to form a ring.

5. The carbon dioxide absorbing composition of any one of the preceding claims, wherein the carbon dioxide absorbing composition further comprises water.

6. The carbon dioxide absorbing composition of any one of the preceding claims, wherein the carbon dioxide absorbing composition further comprises one or more solvents other than water.

7. The carbon dioxide absorbing composition of any one of the preceding claims, wherein the carbon dioxide absorbing composition has a carbon dioxide absorption equivalent represented by the following Equation 1 of 0.6 or more: Carbon dioxide absorption equivalent = (moles of absorbed carbon dioxide) / (moles of ionic material in absorbing composition)

8. The carbon dioxide absorbing composition of any one of the preceding claims, wherein the carbon dioxide absorbing composition further comprises one or more amine absorbents.

9. A method of absorbing carbon dioxide comprising:
reacting carbon dioxide and a phenolate-based anion or an imidazolate-based anion to respectively form a carbonate or a carbamate in an ionic material with a cyclic ammonium cation, thereby obtaining a carbon dioxide absorbing composition, and
additionally physically absorbing carbon dioxide in the carbon dioxide absorbing composition including the ionic material.

10. The method of absorbing carbon dioxide of claim 9, wherein reacting carbon dioxide involves the cyclic ammonium cation to form the ionic material by following the Reaction Formula 1 to form the carbonate and Reaction Formula 2 to form the carbamate respectively specified below:
[Reaction Formula 1]: CO₂ + [heterocycle-N⁺][phenolate-O⁻]→[heterocycle-N⁺][phenolate-O-CO₂⁻]
[Reaction Formula 2]: CO₂ + [heterocycle-N⁺] [imidazolate-N⁻] → [heterocycle-N⁺][imidazolate-N-CO₂⁻],
wherein [heterocycle-N⁺] denotes the cyclic ammonium cation, [phenolate-O⁻] denotes the phenolate-based anion, and [imidazolate-N⁻] denotes the imidazolate-based anion.

11. The method of absorbing carbon dioxide of claim 9 or 10, wherein the carbon dioxide absorbing composition is as defined in any one of claims 1 to 8.

12. A method of separating carbon dioxide, the method comprising:
a first step of bringing the carbon dioxide absorbing composition of any one of claims 1 to 8 into contact with a mixture comprising carbon dioxide;
or carrying out the method of absorbing carbon dioxide of any one of claims 9 to 11;
and
a second step of heat treating the carbon dioxide absorbing composition to desorb carbon dioxide attached to the absorbing composition.

13. The method of separating carbon dioxide of claim 12, wherein the first step is performed under conditions of 20 to 80°C.

14. The method of separating carbon dioxide of claim 12 or 13, wherein the second step is performed under conditions of 70 to 150°C.

15. The method of separating carbon dioxide of any one of claims 12 to 14, wherein when the first step and the second step are a unit process, carbon dioxide is continuously separated by repeating the unit process twice or more.
